Europäisches Patentamt

European Patent Office

Office européen des brevets

(11) Publication number: **0 157 275 B1**

## (12) EUROPEAN PATENT SPECIFICATION

(45) Date of publication of patent specification: **03.01.90**

(21) Application number: **85103132.8**

(22) Date of filing: **18.03.85**

(51) Int. Cl.⁵: **C 12 N 1/16, C 12 N 15/00 // C12R1/865**

(54) Method for culturing transformed yeast.

(30) Priority: **19.03.84 JP 53069/84**

(43) Date of publication of application:
**09.10.85 Bulletin 85/41**

(45) Publication of the grant of the patent:
**03.01.90 Bulletin 90/01**

(84) Designated Contracting States:
**CH DE FR GB LI**

(56) References cited:
**EP-A-0 073 657**
**PROCEEDINGS OF THE NATIONAL ACADEMY OF SCIENCES OF THE USA, vol. 80, no. 1, Jan 1983, Baltimore, MD (US); A.MIYANOHARA et al.: "Expression of hepatitis B surface antigen gene in yeast", pp. 1-5**
**NATURE, vol. 298, no. 5872, 22 July 1982, New York-London; P.VALENZUELA et al.: "Synthesis and assembly of hepatitis B virus surface antigen particles in yeast", pp. 347-350**

(73) Proprietor: **Juridical Foundation The Chemo-Sero-Therapeutic Research Institute**
**668, Okubo Shimizu-machi**
**Kumamoto-shi Kumamoto-ken (JP)**

(72) Inventor: **Muraoka, Toyoharu**
**7-13, Hanazono 3-chome**
**Kumamoto-shi Kumamoto-ken (JP)**
Inventor: **Kudo, Kazusuke**
**2695-21, Suya Nishigoshi-machi**
**Kikuchi-gun Kumamoto-ken (JP)**
Inventor: **Shiosaki, Kou-ichi c/o Juridical Foundation**
**The Chemo-Sero-Therapeutic Res Inst 668, Okubo**
**Shimizu-machi Kumamoto-shi, Kumamoto-ken (JP)**
Inventor: **Mizokami, Hiroshi**
**1647-151, Oaza-Kikudomi Koshi-machi**
**Kikuchi-gun Kumamoto-ken (JP)**

(74) Representative: **Vossius & Partner**
**Siebertstrasse 4 P.O. Box 86 07 67**
**D-8000 München 86 (DE)**

Courier Press, Leamington Spa, England.

**Description**

The present invention relates to a method for culturing a transformed yeast. More particularly, it relates to a method for culturing a yeast transformed by a recombinant plasmid which is recombined with a hepatitis B virus gene and a method for producing hepatitis B virus surface antigen (hereinafter referred to as "HBs antigen" or "HBsAg") by culture of a transformed yeast.

Hepatitis B is a disease which is induced by a hepatitis B virus (hereinafter referred to as "HBV") and includes immunologically and clinically very serius problems, but there has never been found any effective therapeutic method therefor. This disease has been observed worldwidely, but particularly in Asia and Africa.

Against the disease, prophylactic methods have mostly been studied. Suitable prophylaxis is a method of applying a vaccine comprising as an effective ingredient HBs antigen to persons who are afraid to be infected by HBV. A vaccine has already been in practical use, which is prepared by highly purifying an HBs antigen obtained from blood plasma of latent virus carriers who are usually merely designated as "carrier", and inactivating the purified HBs antigen.

However, the blood-origin vaccine is obtained from blood plasma of HBs antigen-positive persons, and hence, it has various problems for the preparation thereof, such as necessity of a safety test in chimpanzees in order to prove that no infectious factors such as hepatitis B virus or any other blood-origin viruses contaminate the preparation.

It is proposed to produce HBs antigen by a transformed *Escherichia coli* prepared by a DNA recombination technique instead of using human blood as starting material (cf. Japanese Patent First Publication No. 104887/1980). However, according to this method using *E. coli,* the produced HBs antigen is easily decomposed within the *E. coli* cells and further *E. coli* hardly grows due to the produced HBs antigen. Due to such drawbacks, the method gives less productivity of HBs antigen and is not suitable for the production of HBs antigen in a large scale.

It is very recently reported that production of HBs antigen particles by a yeast has been succeeded [cf. Nature, *298,* 347 (1982)]. According to the method disclosed in this literature, an HBs protein encoding gene is recombined downstream of an alcohol dehydrogenase (ADH 1) promoter of an *E. coli*-yeast shuttle vector by utilizing said ADH 1 promoter which is used for production of interferon by a yeast.

Besides, it is reported by Miyanohara et al. (cf. Proc. Natl. Acad. Sci., Usa, *80,* 1 (1983) and Japanese Patent First Publication No. 31799/1984) that an HBs protein encoding gene is recombined downstream of a repressible acid phosphatase promoter of an *E. coli*-yeast shuttle vector by utilizing said repressible acid phosphatase promoter.

The present inventors have intensively studied on an improved method for the production of HBs antigen by a yeast in a large scale and have found that HBs antigen can be effectively obtained by culturing a transformed yeast being capable of producing HBs antigen under specific conditions.

An object of the present invention is to provide an improved method for culturing a yeast transformed by a recombinant plasmid which is recombined with a hepatitis B virus gene. A further object of the invention is to provide a method for production of HBs antigen by the culture of said transformed yeast. More particularly, this invention provides a method for culturing a transformed yeast under specific conditions, said transformed yeast being prepared by recombining an HBs antigen gene downstream of a repressible acid phosphatase promoter in a shuttle vector which can grow in both *E. coli* and a yeast, and transforming a yeast with the recombinant plasmid thus prepared.

There are various problems for practical production of HBs antigen in high efficiency and stably by using a transformed yeast. The productivity of HBs antigen is largely effected by the kinds of medium and culture conditions. For instance, various factors such as optimum temperature and pH, etc. for growth of the yeast; amount of dissolved oxygen; optimum temperature and pH, etc. for production of HBs antigen, or the like must be considered as culturing conditions. The present invention provides an improved method for the production of HBs antigen in an industrial scale with high efficiency and high stability by a recombinant yeast with overcoming the above-mentioned various problems.

The method for the production of HBs antigen by a recombinant yeast of the present invention comprises culturing a yeast transformed by a plasmid recombined with HBV gene downstream of a repressible acid phosphatase promoter of a plasmid to produce HBs antigen, which is characteristic in that a semi-synthetic medium containing yeast extract, an inorganic ammonium salt, an inorganic magnesium salt, and either or both of glucose and saccharose is used as a medium of culturing of the transformed yeast.

The yeast transformed by a plasmid recombined with HBV gene downstream of a repressible acid phosphatase promoter of a plasmid to produce HBs antigen is prepared by a method as disclosed by Miyanohara et al. (cf. Proc. Natl. Acad. Sci., USA, *80,* 1 (1983) and Japanese Patent First Publication No. 31799/1984), which comprises preparing a shuttle vector wherein an HBV gene is bound downstream of a repressible acid phosphatase promoter region in a shuttle vector having a replication initiating region of 2 μ plasmid, a replication initiating region of pBR 322 plasmid, a replication initiating region of a yeast chromosome, a leucine-producing gene of a yeast, an ampicillin-resistance gene of *E. coli*, and a region of a repressible acid phosphatase promoter of a yeast, and introducing the shuttle vector into a yeast [AH 22 (a, leu2, his4, Can1, Cir⁺)] or [AH 22 *pho80* (a, leu2, his4, Can1, Cir⁺)] to prepare a transformed yeast.

According to the present invention, the culture of a transformed yeast in a semi-synthetic medium can be carried out by firstly subjecting the transformed yeast to a seed culture in a synthetic medium containing histidine (20 µg/ml) (i.e. BurkHolder minimal medium) and then subjected to main culture, whereby the yeast is grown to produce the desired HBs antigen. When the main culture is carried out in a synthetic medium, it has disadvantageously the following problems.

(1) yeast grows very slowly in a synthetic medium (it usually takes about 72 hours until the stationary phase) and the number of grown cells is so small (e.g. cell number at the stationary phase: $2 \times 10^7 — 3 \times 10^7/1$ ml of culture medium). Accordingly, the production of HBs antigen is so small (e.g. $0.2 — 0.3$ µg/1 ml of culture medium).

(2) It is usually effective to increase the amount of phosphorus in the medium in order to increase the rate and amount of growth of microorganisms. However, in case of the transformed yeast AH 22 as mentioned above, the repressible acid phosphatase promoter does not act in the presence of phosphorus, and hence, the desired HBs antigen can not be produced. Thus, it is required to exchange the medium to a synthetic medium containing no phosphorus at the middle stage of the culture. Moreover, in case of a transformed yeast AH 22 *pho80,* the promoter can act even in the presence of phosphorus, but the productivity of HBs antigen is still very small (cf. Miyanohara et al., Proc. Natl. Acad. Sci., USA, *80,* 1 (1983)].

The semi-synthetic medium used in the present invention contains yeast extract, an inorganic ammonium salt, an inorganic magnesium salt, and either or both of glucose and saccharose, and preferably contains further other additives such as proline and a nonionic surfactant, e.g. polyoxyethylene polyoxypropylene ether. Suitable examples of the inorganic ammonium salt are ammonium sulfate, ammonium chloride, etc. Suitable examples of the inorganic magnesium salt are magnesium sulfate, magnesium chloride, etc. The polyoxyethylene polyoxypropylene ether surfactant includes any commercially available surfactants such as Pluronic (manufactured by Asahi Denka, Japan).

The semi-synthetic medium contains preferably the components in the following concentrations: yeast extract, 0.3—1,0 W/V %; an ammonium salt: 0.5—1.0 W/V %; a magnesium salt: 0.01—0.1 W/V %; saccharose and/or glucose: 2—4 W/V %; proline: 0.01—0.1 mg/ml; and a surfactant: 5—50 ppm by weight.

The semi-synthetic medium contains an appropriate amount of yeast extract, and hence, the yeast can grow in a high growing rate in the presence of the nutrient, phosphorus, and becomes to the stationary phase in about 24 hours, and at that time, the growing amount of cells reaches about $1 \times 10^8$, which is greatly larger than that in case of using a synthetic medium. Besides, since the amount of yeast extract is regulated in a suitable amount, phosphorus is consumed at the logarithmic growth phase of the yeast, which results in that the medium contains substantially no phosphorus. As the result, in either case of the transformed yeast AH 22 and AH *pho80,* the repressible acid phosphatase promoter acts to produce effectively the desired HBs antigen in a large amount.

Other components, ammonium salt, magnesium salt and saccharides (e.g. glucose and/or saccharose) are essential for the growth of the transformed yeast and for the expression of HBs antigen, and when any one of these components lacks, the growth of microorganism and the expression of HBs antigen are remarkably inhibited, but on the other hand, when these components are incorporated in a suitable amount, the productivity of HBs antigen is remarkedly promoted. Besides, although proline and surfactants are not essential components, the addition of these components can promote more the productivity of HBs antigen.

When the amount of yeast extract in the semi-synthetic medium is too large, phosphorus is contained in a too excess amount, which results in lowering of productivity of HBs antigen, and further, due to leucine contained in the yeast extract, a recombinant plasmid having a leucine-producing marker is disadvantageously deleted from the yeast. Thus, the yeast extract should not be incorporated in a too large amount.

The semi-synthetic medium used in the present invention may optionally be incorporated with any other conventional nutrients which are usually incorporated in synthetic media, but such an incorporation does not necessarily result in increase of the production of HBs antigen, but occasionally results in promotion of expression of other characters of the yeast gene and then results disadvantageously in decrease of productivity of HBs antigen.

The method of the production of HBs antigen by culturing of a transformed yeast being capable of producing HBs antigen of the present invention is further characterised in that it is carried out under the conditions at the first stage of the logarithmic growth phase of cells: the culture temperature being 25 to 30°C; the pH of the medium being in the range of 4 to 5.5; and the dissolved oxygen being kept in the range of 2 to 5 ppm by volume; and the conditions at the middle and subsequent stages of the logarithmic growth phase of the cells: the culture temperature being 20 to 24°C; the pH of the medium being in the range of 6.0 to 7.0; and the dissolved oxygen being kept in the range of 2 to 5 ppm by volume.

The above optimum conditions have been confirmed by repeated experiments. For instance, the most suitable culture temperature for showing the highest growth of cells is in the range of about 27 to 28°C, but that for producing the largest amount of HBs antigen is in the range of 22 to 24°C. Thus, the suitable temperature ranges are somewhat different between that for growth of cells and that for expression of HBs antigen. Besides, the most suitable pH for showing the highest growth of cells is in the range of about 4.5 to 5.5, but that for producing the largest amount of HBs antigen is in the range of about 6.0 to 6.5. Thus, the suitable pH ranges are also different between that for growth of cells and that for expression of character of HBs antigen.

Thus, it is effective for growth of cells and for production of HBs antigen by the grown cells to change the temperature and pH ranges between the first stage of the logarithmic growth phase and the middle and subsequent stages of the logarithmic growth phase, which have experimentally been confirmed. It has also experimentally been confirmed that the optimum amount of dissolved oxygen is in the range of 2 to 5 ppm by volume both for growth of cells and for production of HBs antigen.

As is explained above, the present invention can give the desired HBs antigen in high efficiency by culturing a yeast transformed by a recombinant plasmid wherein an HBV gene is inserted into a plasmid having a repressible acid phosphatase promoter of a yeast under controlling of said promoter by using a semi-synthetic medium containing yeast extract, an inorganic ammonium salt, an inorganic magnesium salt and saccharides (e.g. glucose and/or saccharose). Said semi-synthetic medium can easily be prepared in a low cost and is stable even by heat sterilization treatment. The present invention is also advantageous from this viewpoint. Moreover, in combination of the optimum culture conditions for the growth of transformed yeast and for expression of HBs antigen and the use of a semi-synthetic medium, the desired HBs antigen can be obtained in such a high productivity as 0.5 to 0.7 μg per 1 ml of the culture medium (measured by a radioimmunoassay method using AUSRIA—II). Thus, the method of the present invention is very useful for the industrial production of HBs antigen of a yeast and also of hepatitis B vaccine from HBs antigen.

The present invention is illustrated by the following Reference Examples and Examples but should not be construed to be limited thereto.

### Reference Example

In accordance with a method by Miyanohara et al. (cf. Japanese Patent First Publication No. 31799/1984), a transformed yeast being capable of producing HBs antigen is prepared.

### (1) Preparation of HBV DNA
### (i) Preparation of virus DNA

A pooled blood plasma (700 ml) obtained from ten persons (subtype adr) who are positive in HBsAg and HBeAg is centrifuged at 5,000 r.p.m. for 20 minutes to remove undissolved materials. The resulting solution is centrifuged at 4°C, 18,000 r.p.m. for 8 hours, and the resultant precipitates are re-dissolved in 10 ml of a buffer (pH 7.5) of 10mM Tris-HCl, 0.1 M NaCl and 1mM EDTA. The solution is added to the top of a centrifugal tube containing 30% sucrose, which is centrifuged at 4°C, 39,000 r.p.m. for 4 hours. The resultant precipitates are re-dissolved in the same buffer as above.

The buffer solution is subjected to the reaction by HBV DNA polymerase by treating it in a mixture (500 μl) of 67 mM Tris-HCl (pH 7.5), 80mM $NH_4Cl$, 25 mM $MgCl_2$, 0.5% (W/V %, hereinafter, the same) NP40 (tergitol, manufactured by Sigma Co.), 0.1% 2-mercaptoethanol, 330 μM dCTP (deoxycytidine triphosphate), dGTP (deoxyguanosine triphosphate), and dATP (deoxyadenosine triphosphate), 0.5 μMa-[$^{32}$P]dTTP (deoxythymidine triphosphate) at 37°C for 30 minutes. To the reaction mixture is added dTTP in a final concentration of 330 μM, and the mixture is further reacted at 37·C for 3 hours, and to the reaction mixture is added the same volume of 100 mM EDTA solution. By the above DNA polymerase reaction, single-stranded region of the HBV DNA is repaired to wholly double-strand to give a [$^{32}$P] labeled material. This material is added to the top of a centrifugal tube wherein 30%, 20% and 10% aqueous solutions of sucrose are layered in this order, and it is centrifuged at 4°C, 39,000 r.p.m. for 4.5 hours.

In order to digest the proteins strongly bonded to DNA, the precipitates obtained above are treated in a mixture (200 μl) of 1 mg/ml of pronase E (manufactured by Kaken Kagaku K.K.) and 0.2% aqueous sodium lauryl) sulfate solution at 37°C for 2 hours. The resulting mixture is extracted with phenol (200 μl) twice, and the resulting DNA-containing extract is washed with ether to remove phenol solvent to give a solution of HBV DNA. The DNA thus obtained has a specific radioactivity of $2.5 \times 10^6$ cpm/μg and can be used for digestion with restriction enzymes.

### (ii) Cloning of HBV DNA

The double-stranded circular HBV DNA obtained above is cloned by using λ-phage Charon 16A DNA as a vector and then is again cloned by using the known plasmid pACY177 as a vector as follows.

### (A) Cloning on the system of λ-phage Charon 16A host-vector:

HBV DNA (20 ng) is treated with endonuclease Xho I in a mixture (20 μl) of 10 mM Tris-HCl (pH 7.4), 7 mM $MgCl_2$, 100 mM NaCl and 7 mM 2-mercaptoethanol at 37°C for 2 hours. The resulting mixture is extracted with phenol (20 μl) and further with ether, and to the aqueous layer is added a double volume of cooled ethanol to precipitate DNA. The mixture is kept at −70°C for one hour and then centrifuged at 10,000 r.p.m. for 5 minutes, and the precipitated DNA is recovered. The precipitates thus separated are dissolved in a mixture (5 μl) of 10 mM Tris-HCl (pH 7.4) and 1 mM EDTA. The HBV DNA and an equimolar amount of λ-phage Charon 16 A DNA (having one recognition site of Xho I) obtained by cleavage with endonuclease Xho I in the same manner as above are reacted with T4 DNA ligase [a mixture (10 μl) of 50 mM Tris-HCl (pH 7.4), 10 mM $MgCl_2$, 10 mM dithiothreitol, 100 μg/ml calf serum albumin, 0.5 mM ATP and 0.5 μl enzyme

preparation (T4 ligase, manufacturd by Takara Biomedicals, $1—5\times10^3$ unit/ml)] at 4°C for 18 hours. The reaction mixture is extracted with phenol and ether and then subjected to precipitation with ethanol in the same manner as described above. The precipitates thus obtained are dissolved in a mixture (10 μl) of 10 mM Tris-HCl (pH 7.4) and 1 mM EDTA.

The thus annealed DNA is subjected to in vitro packaging operation to form λ-phage in the same manner as described in "Methods in Enzymology", *68*, 299—309 and further plaques ($10^4$) are formed therefrom on an L-agar plate (23 cm × 23 cm) by using *E. coli* DP50 (SupF (cf. Blattner, F.R. et al, Science *196*, 161, 1977) as an indicator. These plaques are subjected to plaque hybridization using $^{32}$P-labeled HBV DNA prepared above as a probe (cf. Science, *196*, 180, 1977) in order to select plaques formed from the phage having HBV DNA, by which a plural of the desired phages are separated.

(B) Re-cloning by using plasmid pACYC177 as a vector:

From the phage having HBV DNA obtained in the above (A), a phage DNA is prepared by using *E. coli* DP50—SupF as a bacteria to be infected in the same manner as described in "Methods in Enzymology", *68*, 245—378, 1979. The DNA thus obtained is digested with Xho I under the same conditions as described above for 2 hours, and the resulting reaction mixture is subjected to an electrophoresis with 0.75% agarose gel to isolate HBV DNA (3.2 kb). The HBV DNA is adsorbed onto DEAE (diethylaminoethyl cellulose) paper (manufactured by Toyo Roshi, Japan) in order to separate from the vector DNA and then eluted with 1 M NaCl aqueous solution to give an HBV DNA having Xho I terminals at both ends.

Separately, plasmid pACYC177 (cf. Chang, A.C.Y., Cohen, S. N.; J. Bacteriol., *134*, 1141—1156, 1978) having a single Xho I cleavage site within kanamycin-resistant gene thereof is digested with Xho I, and the product is purified by phenol extraction, ether treatment and ethanol precipitation in the same manner as described above.

The thus obtained pACYC177 cleaved with Xho I is mixed with Xhol-terminal HBV DNA obtained in a molar ratio of 1:5, and covalently joined by a T4 DNA ligase-catalyzed reaction for 18 hours as described above.

The reaction mixture (10 μl) is added to 0.1 ml of *E. coli* χ1776 [cf. R. III. Curtiss, et al, "Molecular cloning of recombinant DNA" eds. W. A. Scott and R. Werner, page 99, Academic Press (1977)] which is prepared by the procedure as described in M.V. Norgard, Gene, *3*, 279 (1978), and the mixture is mixed well and allowed to stand at 0°C for 25 minutes. The mixture is applied onto an L-agar plate containing ampicillin (20 μg/ml), α-biotine (1 μg/ml), diaminopimelic acid (100 μg/ml) and thymine (20 μg/ml) and is incubated at 37°C overnight. The resulting colonies are applied onto both an agar plate containing kanamycin (20 μg/ml) and an agar plate containing ampicillin (20 μg/ml), and the colonies which grow only on the agar plate containing ampicillin is selected. From the colonies thus selected, a plasmid is prepared by the procedure as described by K. Matsubara (J. Virol., *16*, 479, 1975). The plasmid thus obtained, i.e. the recombinant DNA of pACYC177—HBV DNA (whch is designated "pHBV"), is treated with Xho I under the same conditions as described above to give total HBV DNA fragment (3.2 kb). Besides, when the recombinant DNA is treated with Xho I and Bam HI, there is obtained a fragment (about 1.3 kb) containing HBsAg gene.

(2) Preparation of shuttle vectors pAM 81, pAM 82, pAM 83 and pAM 84.

An EcoRI fragment of about 8,000 nucleotide pair (8 Kb) containing a polypeptide (P60) gene of 60,000 dalton which constitutes the repressible acid phosphatase (RAP) (available from Yeast S288C gene bank; cf. Clarke, L. and Carbon, J., Cell, *9*, 91—99, 1976) is inserted into the EcoRI site of known *E. coli* plasmid pBR322 to give a plasmid, which is used as the starting material.

To remove coding sequence of RAP, the starting plasmid is digested with a restriction enzyme Sal I and covalently joined again with T4 DNA ligase. The resulting plasmid pAT25 is deficient from the Sal I site to the acid phosphatase gene fragment 5.2 kb [said plasmid pAT 25 being a plasmid consisting of a fragment (about 3.7 kb) of from EcoRI site to Sal I site of pBR322 which contains the ampicillin-resistant gene and a fragment (about 2.8 kb) of from EcoRI site to Sal I site of the yeast acid phosphatase gene, wherein both fragments link at each corresponding terminal thereof].

Into the EcoRI site of the above pAT 25 is inserted an EcoRI fragment (1.4 kb) containing ars 1 and Trp 1 gene which is prepared by treating a plasmid YRP 7 (cf. Struhl, K. et al, Proc. Natl. Acad. Sci. U.S.A., *76*, 1035—1039, 1979) with EcoRI to give a plasmid pAT 26. Said ars 1-Trp 1 fragment has a single recognition site of a restriction enzyme Hind III within the Trp 1 gene.

Into the Hind III site of the above pAT 26 is inserted a Hind III fragment containing a Leu 2 and 2 μori which is prepared by treating a plasmid pSLE 1 (cf. Tohe, A. et al, J. Bacteriol, *141*, 413—416, 1980) with Hind III to give a shuttle vector pAT 77. The pAT 77 carried on *Saccharomyces cerevisiae* (i.e. Saccharomyces cerevisiae AH 22/pAT 77) has been deposited at Fermentation Research Institute, Agency of Industrial Science and Technology, Japan under Budapest Treaty as "FERM BP—324".

The pAT 77 thus obtained (1 μg) is cleaved with Sal I and then is treated with an exonuclease BAL 31 (0.1 U) in a solution (50 μl) of 20 mM Tris-HCl (pH 8.2), 12 mM $CaCl_2$, 12 mM $MgCl_2$, 0.2 M NaCl and 1 mM EDTA for 30 seconds to one minute. The reaction mixture is subjected to phenol extraction and ethanol precipitation in the same manner as described above. The resulting precipitates are mixed with Xho I linker (1 pmol) and joined by T4 DNA ligase under the same conditions as described above for 12 hours.

*E. coli* χ1776 is treated with the above reaction mixture by the procedure as described in R. III. Curtiss et al, "Molecular cloning of recombinant DNA" eds. W. A. Scott and R. Werner, page 99, Academic Press (1977) so as to transform the *E. coli* χ1776 to give an ampicillin-resistant transformant. From the resulting transformants, plasmid DNAs are prepared by the procedure as described by K. Matsubara (J. Virol., *16,* 479, 1975). According to Maxam-Gilbert method (cf. Maxam, A. & Gilbert, W.; Proc. Natl. Acad. Sci., *74,* 560—564), the nucleotide sequence of the resulting DNAs is determined, and further, the region of the acid phosphatase gene deleted with BAL 31 is determined. Among these DNAs, the desired plasmids pAM 81, pAM 82, pAM 83 and pAM 84 which are completely deficient in whole of the structural gene of phosphatase are selected and isolated.

Designating "A" in the codon ATG encoding the first amino acid (methionine) of the product P60 of the phosphatase structural gene as "+1", the following regions are deleted in these shuttle vectors, pAM 81: till +2, pAM 82: till −33, pAM 83: till −50, and pAM 84: till −51. The pAM 81, pAM 82, pAM 83 and pAM 84 carried on *Saccharomyces cerevisiae* (i.e. *Saccharomyces cerevisiae* AH 22/pAM 81, AH 22/pAM 82, AH 22/ pAM 83 and AH 22/pAM 84, respectively) have been deposited at Fermentation Research Institute, Agency of Industrial Science and Technology, Japan under Budapest Treaty as "FERM BP—325", "FERM BP—313", "FERM BP—327", and "FERM BP—326", respectively.

(3) Preparation of HBsAg gene-expression plasmids

(i) Preparation of plasmids inserted with whole of HBV DNA

HBV DNA obtained by treating a plasmid pHBV (pACYC 177—HBV DNA) with Xho I is recombined with Xho I cleaved shuttle vector, pAM 81, pAM 82, pAM 83 and pAM 84 in the molar ratio of 5:1 by T4 DNA ligase under the same conditions as described above.

*E. coli* χ1776 is transformed with the reaction mixture and a plasmid DNA is prepared from the resulting ampicillin-resistant transformant in the same manner as described hereinbefore. The DNAs thus prepared are analyzed with various restriction enzymes, such as Xho I, Xba I and Hind III, and thereby, insertion of HBV DNA into the vectors and direction thereof are determined.

The thus obtained HBsAg gene-expression plasmids have HBs gene and HBc gene in this order downstream the phosphatase promoter, and the plasmids recombined with the shuttle vectors, pAM 81, pAM 82, pAM 83 and pAM 84 are designated pAH 201, pAH 203, pAH 205 and pAH 207, respectively.

(ii) Preparation of plasmid inserted with HBsAg gene fragment

An HBsAg gene fragment (3 μg) prepared by cleaving plasmid pHBV with BamHI is treated with T4 DNA polymerase (0.2 U) in a solution (100 μl) of 67 mM Tris-HCl (pH 8.6), 6.7 mM $MgCl_2$, 10 mM 2-mercaptoethanol, 6.7 μM EDTA and 16.7 mM $(NH_4)_2SO_4$ which contains 200 μM αATP, αCTP, αTTP and αGTP for 30 minutes in order to fill-in the BamHI cleavage end. The reaction mixture is subjected to phenol extraction and ethanol precipitation as described above. The resulting precipitates are subjected to linking reaction with Xho I linker in a molar ratio of 1:10 with T4 DNA ligase under the same conditions as described hereinbefore. After phenol extraction and ethanol precipitation, the resulting plasmid is treated with Xho I to give an HBsAg gene fragment (about 1.3 kb) having Xho I cleavage terminal at both ends. The fragment thus obtained is annealed with the shuttle vector pAM 82 which is cleaved with Xho I in a molar ratio of 5:1 by using T4 DNA ligase, and *E. coli* χ1776 is transformed with the reaction mixture obtained above in the same manner as described in the above (i) to give a plasmid DNA.

The plasmid thus obtained is inserted with HBsAg gene in a correct direction downstream the phosphatase promoter of the vector pAM 82, which plasmid is designated pAS 101.

(4) Peparation of transformed yeast

The starting yeast is *Saccharomyces cerevisiae* AH22 [a, leu2, his4, canl (Cir⁺)], which has been deposited at Fermentation Research Institute, Agency of Industrial Science and Technology, Japan under Budapest Treaty as "FERM BP—312". The starting yeast is inoculated in YPD medium (100 ml) consisting of 2% polypeptone, 1% yeast extract and 2% glucose, and the mixture is incubated at 30°C overnight, and thereafter, the cells are collected by centrifugation. The cells thus collected are washed with sterilized water (20 ml), suspended in a solution (5 ml) of 1.2 M sorbitol and 100 μg/ml zymolyase-60,000 (manufactured by Seikagaku Kogyo K.K., Japan), and the suspension is allowed to stand at 30°C for 30 minutes to give spheroplast. The spheroplast thus prepared is washed with 1.2 M sorbitol solution three times, and then suspended in a solution (0.6 ml) of 2 M sorbitol, 10 mM $CaCl_2$ and 10 mM Tris-HCl (pH 7.5). The suspension thus prepared is divided into a small test tube in a volume of 60 μl. To the suspension is added the solution of the recombinant plasmid pAH 203 (30 μl) prepared in the above (3). After mixing well, 0.1 M $CaCl_2$ (3 μl) is added thereto in a final concentration of 10 mM $CaCl_2$, and the mixture is allowed to stand at room temperature for 5 to 10 minutes. To the resulting mixture is added each 1 ml of a solution of 20% polyethylene glycol 4,000, 10 mM $CaCl_2$ and 10 mM Tris-HCl (pH 7.5), and the mixture is allowed to stand at room temperature for about 20 minutes. The resulting mixture (each 0.2 ml) is added to a medium (10 ml) consisting of 22% sorbitol, 2% glucose, 0.7% yeast nitrogen base amino acid, 2% YPD, 20 μg/ml histidine and 3% agar, which is kept at a constant temperature of 45°C. After gently mixing, the mixture is added in a layer onto a plate of minimal medium containing 1.2 M sorbitol which is previously prepared and consists of 0.7% yeast nitrogen base amino acid, 2% glucose, 20 μg/ml histidine and 2% agar and is set thereon. The plate is incubated at 30°C to give a colonie of a leucine-non-requiring yeast. The colonie is incubated in a BurkHolder minimal medium supplemented with histidine (20 μg/ml) [cf. Tohe, A, et al; J. Bachterol., *113,* 727—738, 1973] to give the desired transformed yeast: *Saccharomyces cerevisiae* AH 22/pAH 203.

In the same manner as described above, except that the recombinant plasmids, PAS 101, pAH 201 and pAH 205 are used instead of the recombinant pAH 203, the following transformed yeasts are prepared:

*Saccharomyces cerevisiae* AH 22/pAS 101
*Saccharomyces cerevisiae* AH 22/pAH 201
*Saccharomyces cerevisiae* AH 22/pAH 205

The above procedure is repeated except that there is used *Saccharomyces cerevisiae* AH 22 *pho88 [a leu2 his4 Can1 (Cir⁺)]* (which has been deposited at Fermentation Research Institute, Agency of Industrial Science and Technology, Japan under Budapest Treaty as "FERM BP—509") instead of *Saccharomyces cerevisiae* AH 22 to give the following transformed yeasts:

*Saccharomyces cerevisiae* AH 22 *pho80*/pAS 101
*Saccharomyces cerevisiae* AH 22 *pho80*/pAH 201
*Saccharomyces cerevisiae* AH 22 *pho80*/pAH 203
*Saccharomyces cerevisiae* AH 22 *pho80*/pAH 205

Example 1

The transformed yeast *Saccharomyces cerevisiae* AH 22/pAH 203 obtained in the above Reference Example is inoculated into several tubes which contain each BurkHolder minimal medium (10 ml) supplemented with histidine (20 µg/ml) and incubated at 30°C for 48 hours.

The cultures in three tubes (each 10 ml) are combined and mixed into a medium which is prepared by sterilizing an aqueous solution (3 liters) containing yeast extract (0.5 W/V %), saccharose (4 W/V %), ammonium sulfate (0.5 W/V %), and magnesium sulfate (0.05 W/V %) in an autoclave at 121°C for 15 minutes. The mixture is cultured in a 5 liter jar fermenter under the conditions: temperature, 22°C; pH 6.2—6.5 (controlled); rate of stirring, 300 rpm; dissolved oxygen, 3 ppm (measured by a meter for dissolved oxygen, it is controlled by regulating air blowing) for 36 hours. By the above culture, the yeast grows in 5.8 $\times$ 10$^7$ cells/l ml of culture medium (measured by Coulter Counter®).

The culture medium (10 ml) obtained above was centrifuged to collect the cells and the cells were fractured by glass beads fracturing machine to extract HBs antigen. The content of HBs antigen was measured by a radioimmunoassay method using AUSRIA—II (manufactured by Dainabbott, Japan) wherein a standard purified product of Human blood-origin HBs antigen was used as a reference. As a result, HBs antigen was contained in an amount of 0.16 µg/l ml of the culture medium.

Example 2

The transformed yeast *Saccharomyces cerevisiae* AH 22 *pho80*/pAS 101 obtained in the above Reference Example is inoculated into several tens of tubes which contain each BurkHolder minimal medium (10 ml) supplemented with histidine (20 µg/ml) and incubated at 30°C for 48 hours.

The cultures in three tubes (each 10 ml) are combined. The mixture is each added to 5 jar fermenters which contain each a sterilized medium (3 liters) containing yeast extract (0.5 W/V %), saccharose (4 W/V %), ammonium sulfate (0.5 W/V %), and magnesium sulfate (0.05 W/V %). This mixture is cultured under various conditions as shown in Table 1.

As to the culture thus obtained, the number of grown cells and the amount of the produced HBs antigen were measured in the same manner as in Example 1. The results are shown in Table 1.

7

Table 1

| Run No. | Culture conditions[1] | | | | | | Analytical data[3] | |
|---|---|---|---|---|---|---|---|---|
| | Additives | | Culture temperature (°C) | | pH of culture medium | | Number of cells (cells/ml) | HBs antigen (µg/ml) |
| | Proline (mg/ml) | Detergent[2] (ppm) | Culture hours 0-12hr. | 12-14hr. | Culture hours 0-12hr. | 12-24hr. | | |
| 1 | 0 | 0 | 22 | 22 | 6.2-6.5 | 6.2-6.5 | $5.5 \times 10^7$ | 0.25 |
| 2 | 0 | 0 | 27 | 27 | 4.7-5.0 | 4.7-5.0 | $1.1 \times 10^8$ | 0.28 |
| 3 | 0 | 0 | 27 | 22 | 4.7-5.0 | 6.2-6.5 | $8.7 \times 10^7$ | 0.52 |
| 4 | 0.04 | 10 | 22 | 22 | 6.2-6.5 | 6.2-6.5 | $5.8 \times 10^7$ | 0.43 |
| 5 | 0.04 | 10 | 27 | 22 | 4.7-5.0 | 6.2-6.5 | $8.9 \times 10^7$ | 0.68 |

1) Common conditions: stirring rate, 300 rpm; total culture time, 24 hrs. dissolved oxygen, 3 ppm

2) Pluronic L 61 (manufactured by Asahi Denka K.K.)

3) They were measured in the same manner as described in Example 1.

EP 0 157 275 B1

# EP 0 157 275 B1

## Claims

1. A method for producing HBs antigen by culturing a yeast transformed with a recombinant plasmid which is prepared by inserting a hepatitis B virus gene into a plasmid carrying a repressible acid phosphatase promoter of a yeast, characterized by culturing the transformed yeast in a semi-synthetic medium containing 0.3 to 1.0 w/v% of yeast extract, 0.5 to 1.0 w/v% of an inorganic ammonium salt, 0.01 to 0.1 w/v% of an inorganic magnesium salt, and 2 to 4 w/v% of a saccharide, wherein the culture during the first stage of the logarithmic growth phase of the cells is carried out at a temperature of 25 to 30°C, a pH of the medium in the range of 4 to 5.5, and a content of dissolved oxygen in the range of 2 to 5 ppm by volume, and during the middle and subsequent stages of the logarithmic growth phase of the cells at a temperature of 20 to 24°C. a pH of the medium in the range of 6.0 to 7.0, and a content of dissolved oxygen in the range of 2 to 5 ppm by volume.

2. The method according to claim 1, wherein the inorganic ammonium salt is ammonium sulfate or ammonium chloride, and the inorganic magnesium salt is magnesium sulfate or magnesium chloride.

3. The method according to claim 1, wherein the semi-synthetic medium further contains 0.01 to 0.1 mg/ml of proline, and 5 to 50 ppm of a non-ionic detergent.

4. The method according to claim 3, wherein the non-ionic detergent is a polyoxyethylene polyoxypropylene ether.

5. The method according to claim 1, wherein the saccharide is either or both of glucose and saccharose.

6. The method according to claim 1, wherein the culture during the first stage of the logarithmic growth phase of the cells is carried out at a temperature of 27 to 28°C, a pH of the medium in the range of 4.5 to 5.5, and a content of dissolved oxygen in the range of 2 to 5 ppm by volume, and during the middle and subsequent stages of the logarithmic growth phase of the cells at a temperature of 22 to 24°C, a pH of the medium in the range of 6.0 to 6.5, and a content of dissolved oxygen in the range of 2 to 5 ppm by volume.

## Patentansprüche

1. Verfahren zur Herstellung von HBs-Antigen durch Züchtung einer Hefe, die mit einem rekombinanten Plasmid transformiert ist, welches durch Insertion eines Hepatitis B-Virus-Gens in ein Plasmid, das einen unterdrückbaren Säure Phosphatase-Promotor einer Hefe trägt, hergestellt wird, gekennzeichnet durch Züchtung der transformierten Hefe in einem halbsynthetischen Medium, das 0,3 bis 1,0 Gew./Vol.-% Hefeextrakt, 0,5 bis 1,0 Gew./Vol.-% eines anorganischen Ammoniumsalzes, 0,01 bis 0,1 Gew./Vol.-% eines anorganischen Magnesiumsalzes und 2 bis 4 Gew./Vol.-% eines Saccharids enthält, wobei die Züchtung während der ersten Stufe der logarithmischen Wachstumsphase der Zellen bei einer Temperatur von 25 bis 30°C, einem pH-Wert des Mediums im Bereich von 4 bis 5,5 und einem Gehalt an gelöstem Sauerstoff im Bereich von 2 bis 5 Volumen-p.p.m., und während der mittleren und nachfolgenden Stufen der logarithmischen Wachstumsphase der Zellen bei einer Temperatur von 20 bis 24°C, einem pH-Wert des Mediums im Bereich von 6,0 bis 7,0 und einem Gehalt an gelöstem Sauerstoff im Bereich von 2 bis 5 Volumen-p.p.m. durchgeführt wird.

2. Verfahren nach Anspruch 1, wobei das anorganische Ammoniumsalz Ammoniumsulfat oder Ammoniumchlorid und das anorganische Magnesiumsalz Magnesiumsulfat oder Magnesiumchlorid ist.

3. Verfahren nach Anspruch 1, wobei das halbsynthetische Medium ferner 0,01 bis 0,1 mg/ml Prolin und 5 bis 50 T.p.M. eines nicht-ionischen Detergens enthält.

4. Verfahren nach Anspruch 3, wobei das nicht-ionische Detergens ein Polyoxyäthylen-Polyoxypropylen-Äther ist.

5. Verfahren nach Anspruch 1, wobei das Saccharid entweder Glukose oder Saccharose oder beides ist.

6. Verfahren nach Anspruch 1, wobei die Züchtung während der ersten Stufe der logarithmischen Wachstumsphase der Zellen bei einer Temperatur von 27 bis 28°C, einem pH-Wert des Mediums im Bereich von 4,5 bis 5,5 und einem Gehalt an gelöstem Sauerstoff im Bereich von 2 bis 5 Volumen-p.p.m. und während der mittleren und nachfolgenden Stufen der logarithmischen Wachstumsphase der Zellen bei einer Temperatur von 22 bis 24°C, einem pH-Wert des Mediums im Bereich von 6,0 bis 6,5 und einem Gehalt an gelöstem Sauerstoff im Bereich von 2 bis 5 Volumen-p.p.m. durchgeführt wird.

## Revendications

1. Procédé pour la production d'antigène HBs par culture d'une levure transformée avec un plasmide recombinant, qui est préparé par insertion d'un gène de virus de l'hépatite B dans un plasmide portant un promoteur phosphatase acide répressible d'une levure, caractérisé par la culture de la levure transformée dans un milieu semi-synthétique contenant 0,3 à 1,0% p/v d'extrait de levure, 0,5 à 1,0% p/v d'un sel minéral d'ammonium, 0,01 à 0,1% p/v d'un sel minéral de magnésium et 2 à 4% p/v d'un saccharide, où la culture, pendant le premier stade de la phase de croissance logarithmique des cellules, est effectuée à une température de 25 à 30°C, à un pH du milieu dans la gamme de 4 à 5,5 et à une teneur en oxygène dissous dans la gamme de 2 à 5 ppm en volume et, pendant les stades intermédiaire et ultérieur de la phase de

9 ·

croissance logarithmique des cellules, à une température de 20 à 24°C, à un pH du milieu dans la gamme de 6,0 à 7,0 et à une teneur de l'oxygène dissous dans la gamme de 2 à 5 ppm en volume.

2. Procédé selon la revendication 1, dans lequel le sel minéral d'ammonium est le sulfate d'ammonium ou le chlorure d'ammonium et le sel minéral de magnésium est le sulfate de magnésium ou le chlorure de magnésium.

3. Procédé selon la revendication 1, dans lequel le milieu semi-synthétique contient de plus 0,01 à 0,1 mg/ml de proline et 5 à 50 ppm d'un détergent non ionique.

4. Procédé selon la revendication 3, dans lequel le détergent non ionique est un polyoxyéthylène-poly-oxypropylène-éther.

5. Procédé selon la revendication 1, dans lequel le saccharide est le glucose et/ou le saccharose.

6. Procédé selon la revendication 1, dans lequel la culture, pendant le premier stade de la phase de croissance logarithmique des cellules, est effectuée à une température de 27 à 28°C, à un pH du milieu dans la gamme de 4,5 à 5,5 et à une teneur en oxygène dissous dans la gamme de 2 à 5 ppm en volume et, pendant les stades intermédiaire et ultérieur de la phase de croissance logarithmique des cellules, à une température de 22 à 24°C, à un pH du milieu dans la gamme de 6,0 à 6,5 et à une teneur en oxygène dissous dans la gamme de 2 à 5 ppm en volume.